(19) **Europäisches Patentamt / European Patent Office / Office européen des brevets**

(11) **EP 1 975 617 A1**

(12) **EUROPEAN PATENT APPLICATION**

(43) Date of publication:
**01.10.2008 Bulletin 2008/40**

(21) Application number: **08005791.2**

(22) Date of filing: **27.03.2008**

(51) Int Cl.:
*G01N 33/543* (2006.01)    *G01N 33/553* (2006.01)
*G01N 21/55* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.03.2007   JP 2007091411**

(71) Applicant: **FUJIFILM Corporation**
**Minato-ku**
**Tokyo (JP)**

(72) Inventors:
- **Nishimi, Taisei,**
  **c/o Fujifilm Corporation**
  **Ashigarakami-gun,**
  **Kanagawa 258-8577 (JP)**
- **Kawamura, Koichi,**
  **c/o Fujifilm Corporation**
  **Ashigarakami-gun,**
  **Kanagawa 258-8577 (JP)**

(74) Representative: **HOFFMANN EITLE**
**Patent- und Rechtsanwälte**
**Arabellastrasse 4**
**81925 München (DE)**

(54) **A solid substrate on which a phisiologically active substance immobilized**

(57)    It is an object of the present invention to provide a solid substrate, on which a physiologically active substance is inclusively immobilized, and a production method thereof. The present invention provides a solid substate, which has a physiologically active substance in a crosslinked hydrogd composed of a polysaccharide that was covalently bound to the surface of the solid substrate.

**Description**

TECHNICAL FIELD

The present invention relates to a solid substrate on which a physiologically active substance is immobilized, and a production method thereof. More specifically, the present invention relates to a substrate used for sensors, on which a physiologically active substance is immobilized, which is used in surface plasmon resonance analysis and the like, and a production method thereof.

BACKGROUND ART

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is earned out, thereby detecting a substance associated with a living organism from sedimentation of gold fine particles or sequences.

In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.

As a thin film having a functional group capable of immobilizing a physiologically active substance, a measurement chip, on which a physiologically active substance is immobilized using a functional group binding to metal, a linker having 10 or more atoms as a chain length, and a compound having a functional group capable of binding to the physiologically active substance, has been reported (Japanese Patent No. 2815120). A specific example of such a measurement chip is a CMD (carboxymethyl dextran) surface which is used as a surface for immobilizing a physiologically active substance on an SPR sensor substrate, such as Biacore CM5. However, since a physiologically active substance is immobilized on this surface via a covalent bond (amide bond), there may be cases where inactivation of the immobilized protein is induced.

A method for encapsulating a physiologically active substance in a crosslinked hydrogel is called an "inclusive immobilization method" Such an inclusive immobilization method has been known since the 1970s. For example, JP Patent Publication (Kokai) No. 5-133928 A (1993) describes that a crosslinked hydrogel is formed by applying light to a synthetic polymer used as a hydrogel, such as cinnamoyloxy ethyl methacrylate, and a protein is then inclusively immobilized therein. When this sensor surface is repeatedly used, it is necessary that the surface, on which a physiologically active substance is inclusively immobilized, be not removed from the substrate. However, methods of allowing an inclusive immobilization surface to strongly bind to a sensor surface are limited. Thus, it is desired to develop a generally used method.

DISCLOSURE OF THE INVENTION

It is an object of the present invention to provide a solid substrate, on which a physiologically active substance is inclusively immobilized, and a production method thereof.

As a result of intensive studies directed towards achieving the aforementioned object, the present inventors have found that a physiologically active substance can be immobilized in a crosslinked hydrogel covalently bound to the surface of the solid substrate by allowing an aqueous solution comprising a physiologically active substance, a water-soluble monomer and a water-soluble initiator to come into contact with a solid substrate, to the surface of which a polysaccharide having a double bond or a derivative thereof is bound, and carrying out polymerization. The present invention has been completed based on such findings.

The present invention provides a solid substrate, which has a physiologically active substance in a crosslinked hydrogel composed of a polysaccharide that was covalently bound to the surface of the solid substrate.

The present invention further provides a solid substrate, wherein a physiologically active substance is inclusively immobilized in a crosslinked hydrogel composed of a polysaccharide which was covalently bound to the surface of the solid substrate.

Preferably, the crosslinked hydrogel is formed using a polysaccharide having a double bond or a derivative thereof, a water-soluble monomer, and a water-soluble initiator.

Preferably, the solid substrate of the present invention is produced by allowing an aqueous solution comprising a physiologically active substance, a water-soluble monomer and a water-soluble initiator, to come into contact with the solid substrate, to which a polysaccharide having a double bond or a derivative thereof was bound, so as to carry out polymerization.

Preferably, in the aqueous solution comprising a physiologically active substance, a water-soluble monomer and a water-soluble initiator, the concentration of the water-soluble monomer is between 100 mM and 1 M.

Preferably, in the aqueous solution comprising a physiologically active substance, a water-soluble monomer and a water-soluble initiator, the concentration of the water-soluble initiator is between 0.1 mM and 2 mM.

Preferably, the water-soluble initiator is persulfate.

Preferably, the water-soluble monomer has an amide structure or a polyether structure.

Preferably, the aqueous solution comprising a physiologically active substance, a water-soluble monomer and a water-soluble initiator, further comprises a polymerization promoter.

Preferably, the polymerization initiator is β-dimethylaminopropionitrile, N,N,N',N'-tetramethylethylenediamine, or sodium sulfite.

Preferably, the polysaccharide having a double bond or a derivative thereof is generated as a result of the reaction of a carboxyl group-containing polysaccharide which was covalently bound to the solid substrate or a derivative thereof with an amine having a double bond.

Preferably, the physiologically active substance is a protein.

According to another aspect, the present invention provides a biosensor, which comprises the solid substrate of the present invention as mentioned above.

Preferably, the biosensor of the present invention is used in surface plasmon resonance analysis.

According to further another aspect, the present invention provides a method for producing a solid substrate having a physiologically active substance in a crosslinked hydrogel composed of a polysaccharide that was covalently bound to the surface of the solid substrate, which comprises allowing an aqueous solution comprising a physiologically active substance, a water-soluble monomer and a water-soluble initiator, to come into contact with a solid substrate, to the surface of which a polysaccharide having a double bond or a derivative thereof was bound, so as to carry out polymerization.

Preferably, the polysaccharide having a double bond or a derivative thereof that was bound to the surface of the solid substrate has a film thickness in water of between 5 nm and 500 nm.

In the solid substrate of the present invention, since a physiologically active substance is included in a crosslinked hydrogel, it is possible to immobilize such a physiologically active substance such as a protein on the substrate in a state where the activity of the physiologically active substance can be maintained high. In addition, in the solid substrate of the present invention, since a polysaccharide is used as a constituent of the hydrogel for including the physiologically active substance, it is possible to enhance the effect of suppressing inactivation of the physiologically active substance. Moreover, in the solid substrate of the present invention, since a polysaccharide is immobilized on the solid substrate via a covalent bond, the crosslinked hydrogel does not leak out, even If long-period measurement or washing is carried out Thus, the thickness of the film can be controlled.

BEST MODE FOR CARRYING OUT THE INVENTION

The embodiments of the present invention will be described in detail below.

In the solid substrate of the present invention, a physiologically active substance is contained in a crosslinked hydrogel that was covalently bound to the surface thereof. Herein, a preferred embodiment of the solid substrate of the present invention will be described more in detail. A crosslinked gel is immobilized on the surface of the solid substrate in a state where a physiologically active substance is included in the crosslinked gel. More preferably, a crosslinked hydrogel acts as a birdcage, and a physiologically active substance is immobilized on a substrate surface via such a crosslinked hydrogel. Accordingly, the physiologically active substance does not leak out of the crosslinked gel, and it is immobilized on the substrate surface. Hereinafter, a method for immobilizing a physiologically active substance using the solid surface of the present invention is referred to as "inclusive immobilization."

(1) Substrate

The solid substrate of the present invention can be used as a substrate for biosensor, for example. The biosensor in the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

In the present invention, a metal surface or metal film can be used as a substrate. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above carrier, an interstitial layer consisting of chrome or the like may be provided between the carrier and a metal layer.

The film thickness of a metal film is not limited. When the metal film is used for a surface plasmon resonance biosensor, the thickness is preferably between 0.1 nm and 500 nm, and particularly preferably between 1 mn and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method

The metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate. When a substrate of the present invention is used for a surface plasmon resonance biosensor, examples of a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The aforementioned substrate is immobilized on the dielectric block of a measurement unit and is unified therewith to construct a measurement chip. This measurement chip may be exchangeably formed.

(2) Crosslinked hydrogel

A polysaccharide that is a constituent of a crosslinked hydrogel which can be used in the present invention has a film thickness in water (a film thickness of only the polysaccharide before immobilization of a physiologically active substance) of preferably between 5 nm and 500 nm, and more preferably between 10 mn and 300 nm. If such a film thickness is too thin, the amount of a physiologically active substance immobilized is decreased, and a hydrated layer on the sensor surface becomes thin. As a result, it becomes difficult to detect the interaction of the physiologically active substance with a test substance due to degeneration of the physiologically active substance itself. On the other hand, if such a film thickness is too thick, it prevents the test substance from dispersing into the film, and in particular, when the interaction is detected from the side opposite to the crosslinked hydrogel-immobilized surface of the sensor substrate, the distance from the detection surface to an interaction-forming portion becomes too long, resulting in a low detection sensitivity. The film thickness of a polysaccharide in water can be measured by AFM, ellipsometry, or the like.

In the present invention, by using a polysaccharide as a constituent of a crosslinked hydrogel, non-specific adsorption of contaminants can be suppressed. Examples of such a polysaccharide used in the present invention include gelatin, agarose, chitosan, dextran, carragheenan, alginic acid, starch, cellulose, and a derivative thereof such as a carboxymethyl derivative. More specific examples include hyaluronic acid, chondroitin sulfate, heparin, dermatan sulfate, carboxymethyl cellulose, carboxyethyl cellulose, cellouronic acid, carboxymethyl chitin, carboxymethyl dextran, and carboxymethyl starch. In the present invention, a carboxy group-containing polysaccharide is preferably used. As such a carboxy group-containing polysaccharide, commercially available compounds can be used. Specific examples of such commercially available compounds include: carboxymethyl dextran such as CMD, CMD-L and CMD-D40 (manufactured by Meito Sangyo Co., Ltd.); carboxymethylcellulose sodium (Wako Pure Chemical Industries, Ltd); and sodium alginate (Wako Pure Chemical Industries, Ltd.). The molecular weight of the polysaccharide used in the present invention is not particularly limited. In general, it is between 200 and 5,000,000.

The constituent of the aforementioned crosslinked hydrogel may be immobilized on a substrate via aminopropyltriethoxysilane or a self-assembled membrane. Otherwise, the constituent may be formed on a substrate directly from a solution containing a monomer.

The self-assembled membrane refers to an ultrathin film such as a monomolecular film or LB film which has an organization with certain order formed by a mechanism possessed by a membrane material itself in a state where detailed control is not applied thereto from outside. This self-assembly forms an orderly structure or pattern over a long distance in non-equilibrium conditions. A method for coating a metal film with the use of a self-assembled membrane (SAMs) has been actively developed by Professor Whitesides et al. (Harvard University). Details of the method are reported in, for example, Chemical Review, 105, 1103-1169 (2005). When gold is used as a metal, an orientational self-assembled monomolecular film is formed with the use of an alkanethiol derivative represented by the following formula A-1 (in the formula A-1, n represents an integer from 3 to 20, and X represents a functional group) as an organic layer-forming compound based on the van der Waals force between an Au-S bond and an alkyl chain. A self-assembled membrane is formed by a very simple method, wherein a gold substrate is immersed in a solution of an alkanethiol derivative.

$$HS(CH_2)_nX$$
$$\underline{\text{A-1}}$$

In the present invention, the self-assembled membrane preferably has an amino group. A self-assembled membrane is formed with the use of a compound (represented by the formula A-1 where X is $NH_2$), so that it becomes possible to coat a gold surface with self-assembled membrane having an amino group:

An alkanethiol having an amino group may be a compound comprising a thiol group and an amino group linked via an alkyl chain (formula A-2) (in the formula A-2, a represents an integer of 3 to 20), or may be a compound obtained by reaction between alkanethiol having a carboxyl group at the end (formula A-3 or A-4) (in the formula A-3, n represents an integer of 3 to 20, and in the formula A-4, n each independently represents an integer of 1 to 20) and a large excess of hydrazide or diamine. The reaction between alkanethiol having a carboxyl group at the end and a large excess of hydrazide or diamine may be performed in a solution state. Alternatively, the alkanethiol having a carboxyl group at the end may be bound to the substrate surface and then reacted with a large excess of hydrazide or diamine.

$$HS(CH_2)_nNH_2 \qquad \underline{\text{A-2}}$$

$$HS(CH_2)_nCOOH \qquad \underline{\text{A-3}}$$

$$HS(CH_2)_n(OCH_2CH_2)_nOCH_2COOH \qquad \underline{\text{A-4}}$$

The repeating number of alkyl group of the formulas A-2 to A-4 is preferably 3 to 20, more preferably 3 to 16, and most preferably 4 to 8. If the alkyl chain is short, formation of self-assembled membrane becomes difficult, and if the alkyl chain is long, water solubility decreases and the handling becomes difficult

Any compound may be used as the diamine used in the present invention. An aqueous diamine is preferable for use in the biosensor surface. Specific examples of the aqueous diamine may include aliphatic diamine such as ethylenediamine, tetraethylenediamine, octamethylenediamine, decamethylenediamine, piperazine, triethylenediamine, diethylenetriamine, triethylenetetraamine, dihexamethylenetriamine, and 1,4-diaminocyclohexane, and aromatic diamine such as paraphenylenediamine, metaphenylenediamine, paraxylylenediamine, metaxylylenediamine, 4,4'-diaminobiphenyl, 4,4'-diaminodiphenylmethane, 4,4'-diaminodiphenylketone, and 4,4'4aminodiphenylsulfonic acid. From the viewpoint of increasing the hydrophilicity of the biosensor surface, a compound comprising two amino groups linked via an ethylene glycol unit (formula A-5) may also be used. The diamine used in the present invention is preferably ethylenediamine or the compound represented by the formula A-5 (in the formula A-5, n and m each independently represent an integer of 1 to 20), more preferably ethylenediamine or 1,2-bis(aminoethoxy)ethane (represented by the formula A-5 wherein n=2 and m=1).

$$H_2N(CH_2)_n(OCH_2CH_2)_mO(CH_2)_nNH_2 \qquad \underline{\text{A-5}}$$

The alkanethiol having an amino group may form a self-assembled membrane by itself or may form a self-assembled membrane by mixing it with another alkanethiol. It is preferred for use in the biosensor surface that a compound capable of suppressing the nonspecific adsorption of a physiologically active substance should be used as the another alkanethiol. The aforementioned Professor Whitesides et al. have investigated in detail self-assembled membrane capable of suppressing the nonspecific adsorption of a physiologically active substance and have reported that a self-assembled membrane formed from alkanethiol having a hydrophilic group is effective for suppressing nonspecific adsorption (Langmuir,17, 2841-2850, 5605-5620, and 6336-6343 (2001)). In the present invention, any of compounds described in the aforementioned papers may be used preferably as the alkanethiol that forms a mixed monolayer with an alkanethiol having an amino group. In terms of excellent ability to suppress nonspecific adsorption and ease of acquisition, it is preferred that alkanethiol having a hydroxyl group (formula A-6) or alkanethiol having an ethylene glycol unit (formula A-7) (m the formula A-6, n represents an integer of 3 to 20, and in the formula A-7, n and m each independently represent an integer of 1 to 20) should be used as the alkanethiol that forms a mixed monolayer with an alkanethiol having an amino group.

$$HS(CH_2)_nOH \qquad \underline{A\text{-}6}$$

$$HS(CH_2)_n(OCH_2CH_2)_mOH \qquad \underline{A\text{-}7}$$

When alkane thiol having an amino group is mixed with another alkane thiol to form a self-assembled membrane, the repeating number of alkyl group of the formulas A-2 to A-4 is preferably 4 to 20, more preferably 4 to 16, and most preferably 4 to 10. Further, the repeating number of alkyl group of the formulas A-6 and A-7 is preferably 3 to 16, more preferably 3 to 12, and most preferably 3 to 8.

In the present invention, it is possible to mix alkanethiol having an amino group and alkanethiol having a hydrophilic group at an arbitrary ratio. However, when the content of alkanethiol having an amino group is low, the amount of polysaccharide to be bound decreases. When the content of alkanethiol having a hydrophilic group is low, the capacity for suppression of nonspecific adsorption is reduced. Thus, the mixing ratio of alkanethiol having an amino group to alkanethiol having a hydrophilic group is preferably 1:1 to 1:1,000,000, more preferably 1:4 to 1:10,000, and further preferably 1:10 to 1:1,000. In view of reduction of steric hindrance upon a reaction with an actively esterified carboxyl group-containing polymer, the molecular length of alkanethiol having an amino group is preferably longer than that of alkanethiol having a hydrophilic group.

As alkanethiol used for the present invention, compounds synthesized based on Abstract, Curr. Org. Chem., 8,1763-1797 (2004) (Professor Grzybowski, Northwestern University) and references cited therein or a commercially available compound may be used. It is possible to purchase such compounds from Dojindo Laboratories, Aldrich, SensoPath Technologies, Frontier Scientific Inc., and the like. In the present invention, disulfide compounds that are oxidation products of alkanethiol can be used in the same manner as alkanethiol.

(3) Method for producing a solid substrate

In a method for producing the solid substrate of the present invention, an aqueous solution comprising a physiologically active substance, a water-soluble monomer and a water-soluble initiator, is allowed to come into contact with a solid substrate to the surface of which a polysaccharide having a double bond or a derivative thereof is bound, so as to carry out polymerization, so that a crosslinked hydrogen including the physiologically active substance can be formed.

The polysaccharide having a double bond or a derivative thereof can be generated by allowing a carboxyl group-containing polysaccharide or a derivative thereof which was covalently bound to a solid substrate, to react with an amine having a double bond. Examples of an amine having a double bond that can be used herein include: C3-6 alkenyl amines such as 2-aminoethyl methacrylate, (meth)allylamine, or crotylamine; amino C2-6 alkyl (meth)acrylates such as aminoethyl (meth)acrylate; and monomers having an aromatic ring and a primary amino group, such as vinylaniline. Of these, amino C2-6 alkyl (meth)acrylates are preferable, and aminoethyl methacrylate is more preferable.

As a water-soluble monomer used in the present invention, any one of a nonionic monomer, an anionic monomer, and a cationic monomer can be used. Of these, a nonionic monomer is preferably used.

Examples of the nonionic monomer include those having an amide structure, those having a hydroxyl structure, those having an ester structure, and those having a polyether structure. Among others, those having an amide structure or a polyether structure are preferable. More specific examples of such a preferred nonionic monomer include acrylamide, $C_{1-3}$ N-alkyl or $C_{1-3}$ N,N-dialkylacrylamide, polyethylene glycol acrylate, polyethylene glycol methacrylate, N-vinylacetamide, N-msthyl-N-vinylacetamide, N-vinylformamide, N-methyl-N-vinylformamide, N-vinyl lactam comprising a cyclic group having 4 to 9 carbon atoms, vinyl alcohol (obtained by hydrolysis after polymerization in the form of vinyl acetate), ethylene oxide, hydroxyethyl acrylate, hydroxypropyl acrylade, hydroxyethyl methacrylate, and hydroxypropyl methacrylate, Of these, acrylamide, $C_{1-3}$ N-alkyl or $C_{1-3}$ N,N-dialkylacrylamide, polyethylene glycol acrylate, and polyethylene glycol methacrylate are preferable.

Examples of the anionic monomer include ethylene unsaturated carboxylic acids such as acrylic acid, methacrylic acid, itaconic acid, fumaric acid, crotonic acid, maleic acid, 2-actylamide-2-methylpropanesulfonic acid, styrenesulfonic acid, vinylsulfonic acid, or vinylphosphonic acid.

Examples of the cationic monomer include dimethyldiallylammonium chloride, methylvinylimidazolium chloride, 2-vinylpyridine, 4-vinylpyridine, 2-methyl-5-vinylpyridine, vinylamine, and a monomer represented by the following formula:

$$H_2C = CR_1\text{-}CO\text{-}X_2$$

(wherein $R_1$ represents a hydrogen atom or a methyl group; and $X_2$ represents a linear or branched $C_{1-6}$ hydrocarbon group having at least one primary, secondary or tertiary amine functional group, or at least one quaternary nitrogen atom, or a group represented by the formula $NHR_2$ or the formula $NR_2R_3$, wherein each of $R_2$ and $R_3$ independently represents a linear or branched $C_{1-6}$ hydrocarbon group having at least one primary, secondary or tertiary amine functional group, or at least one quaternary nitrogen atom).

The water-soluble monomers may be used singly, or in the form of a mixture of two or more types of different monomers.

In addition, the water-soluble monomer is preferably adjusted to be a concentration between 10 mM and 10 M in an aqueous solution comprising a physiologically active substance, a water-soluble monomer and a water-soluble initiator. The aforementioned concentration is more preferably between 50 mM and 2 M, and further more preferably between 100 mM and 1 M.

Examples of a water-soluble initiator used in the present invention include a water-soluble persulfate, peroxide, and an azobis compound. Of these, persulfate is preferable. Specific examples of the preferred water-soluble initiator include compounds such as potassium persulfate, sodium persulfate, ammonium persulfate, hydrogen peroxide, t-butyl peroxymaleic acid,
2,2-azobis(2-diaminopropane)dihydrochloride,
2,2-azobis[2-(5-methyl-2-imidazoli-2-nyl)propane]dihydrochloride, 22-azobis[2-(2-imidazoli-2-nyl)propane]dihydrochloride,
2,2-azobis{2-methyl-N-[1,1-bis(hydroxymethyl)-2-hydroxyethyl]propionamide},
2,2-azobis[2-methyl-N-(2-hydroxyethyl)propionamide], or
2,2-azobis(2-methyl-propionamide)dihydrate. Of these, potassium persulfate, sodium persulfate, and ammonium persulfate are more preferable.

In addition, the water-soluble initiator is preferably adjusted to be a concentration between 0.01 mM and 100 mM in an aqueous solution comprising a physiologically active substance, a water-soluble monomer and a water-soluble initiator. The aforementioned concentration is more preferably between 0.05 mM and 10 mM, further more preferably between 0.1 mM and 2 mM, and still further more preferably between 0.1 mM and 1 mM.

It is also preferable that the water-soluble initiator be used together with a polymerization promoter such as β-dimethylaminopropionitrile, N,N,N',N'-tetramethylethylenediamine, or sodium sulfite. When such a polymerization promoter is used together with the water-soluble initiator, the concentration of the polymerization promoter is preferably between 0.1 mM and 1 M, more preferably between 0.5 mM and 100 mM, and further more preferably 1 mM and 10 mM, in an aqueous solution comprising a physiologically active substance, a water-soluble monomer and a water-soluble initiator.

(4) Physiologically active substance which can be used in the present invention

A physiologically active substance which is immobilized in the crosslinked hydrogel in the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, mstaicillin-resistant *Staphylococcus aureus,* antibiotic, narcotic drug, cocaine, heroin, crack or the like, there can be used, for example, an anti-atrazine antibody, anti-kanamycin antibody, ann-metamphetamine antibody, or antibodies against 0 antigens 26, 86, 55,111 and 157 among enteropathogenic *Escherichia coli.*

An enzyme used as a physiologically active substance herein is not particularly limited, as long as it exhibits an activity to a measurement target or substance metabolized from the measurement target Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor.

Examples of an mimunoglobulm-binding protein used herein may include protein A, protein G, and a rheumatoid factor (RF).

As a sugar-binding protein, for example, lectin is used.

Examples of fatty acid or fatty acid ester may include stearic acid, arachidic acid, behenic acid, ethyl stearate, ethyl arachidate, and ethyl behenate.

Among these physiologically active substances, it is preferred to use protein, and it is more preferred to use protein A, protein G, avidins, calmodulin, or antibody.

Moreover, the physiologically active substance is adjusted to a concentration preferably between 0.001 mg/ml and 5 mg/ml, more preferably between 0.005 mg/ml and 1 mg/ml, and further more preferably between 0.05 mg/ml and 0.5 mg/ml, in an aqueous solution comprising a physiologically active substance, a water-soluble monomer and a water-soluble initiator.

Generally, physiologically active substance such as protein maintain its three-dimensional structure by coordination of water molecules in a solution, but when it is dried, physiologically active substance cannot maintain its three-dimensional structure and is denatured. Further, physiologically active substance is contained in a hydrophilic polymer compound on a surface of substrate, physiologically active substance aggregate by drying, and aggregates are produced. The compound (hereinafter referred to as Compound S) having a residue capable of forming hydrogen bond which may be used in the present invention can be used for the purpose of suppressing denature of physiologically active substance by maintaining the three-dimensional structure in place of water or suppressing the aggregation by steric effect by covering the physiologically active substance.

In the present invention, the Compound S having a residue capable of forming hydrogen bond is preferably added as a aqueous solution to a layer on substrate where physiologically active substance was immobilized. Compound S can be added by coating a mixed solution of Compound S and physiologically active substance on a surface of substrate, or by immobilizing physiologically active substances on a surface of substrate and then over-coating the Compound S. When a mixed solution of Compound S and physiologically active substance is coated, fluctuation of the amount of immobilized physiologically active substances can be reduced. Preferably, an aqueous solution of Compound S can be added to substrate in a state of thin film. A method for forming thin film on substrate may be any known method. Examples thereof include extrusion coating, curtain coating, casting, screen printing, spin coating, spray coating, slidebead coating, slit and spin coating, slit coating, die coating, dip coaling, knife coating, blade coating, flow coating, roll coating, wire-bar coating, and transferring printing. In the present invention, spray coating or spin coating is preferably used, and spin coating is more preferably used as a method for forming a thin film on substrate, since a coated film having a controlled film thickness can be easily prepared.

The concentration of the applied solution of compound S is not particularly limited, as long as it does not cause a problem regarding permeation into a layer that contains physiologically active substances. The aforementioned concentration is preferably between 0.1% by weight and 5% by weight. In addition, in terms of applicability and regulation of pH, a surfactant, a buffer, an organic solvent, a salt may also be added to the applied solution.

The compound S having a residue capable of forming hydrogen bond is preferably a compound which is nonvolatile under normal pressure at normal temperature. The average molecular weight of the compound is preferably 350 to 5,000,000, more preferably 1,200 to 2,000,000, most preferably 1,200 to 70,000. The compound S having a hydroxyl group in molecule is preferably saccharide. The saccharide may be monosaccharide or polysaccharide. In case of n-saccharide, n is preferably 4 to 1,200, and n is more preferably 20 to 600.

If the mean molecular weight of compound S is too low, the compound is crystallized on the surface of a substrate. This causes disruption of a hydrophilic polymer layer, on which physiologically active substances are immobilized, and disruption of the three-dimensional structure of the physiologically active substances. In contrast, if the mean molecular weight of compound S is too high, it causes problems such that it impairs immobilization of physiologically active substances on a substrate, that a layer that contains physiologically active substances cannot be impregnated with compound S, and that layer separation occurs.

For the purpose of suppressing degradation of physiologically active substances immobilized on a substrate, the aforementioned compound S having a residue capable of forming hydrogen bond preferably has a dextran skeleton or a polyethylene oxide skeleton. The type of a substituent used is not limited, as long as the object of the present invention can be achieved. Moreover, for the purpose of suppressing degradation of physiologically active substances immobilized on a substrate, a nonionic compound having no dissociable groups is preferably used as compound S. Furthermore, the aforementioned compound S having a residue capable of forming a hydrogen bond preferably has high affinity for water molecules. A distribution coefficient LogP value between water and n-octanol is preferably 1 or greater. Such LogP value can be measured by the method described in Japanese Industrial Standard (JIS), Z7260-107 (2000), "Measurement of distribution coefficient (1-octanol/water) - Shaking method," etc.

Specific examples of compound S having a residue capable of forming hydrogen bond include: compounds consisting of two or more types of residues selected from polyalcohols such as polyvinyl alcohol, proteins such as collagen, gelatin, or albumin, polysaccharides such as hyaluronic acid, chitin, chitosan, starch, cellulose, alginic acid, or dextran, polyethers including polyethyleneoxy-polypropylene oxide condensates such as polyethylene glycol, polyethylene oxide, polypropylene glycol, polypropylene oxide, or Pluronic, Tween 20, Tween 40, Tween 60, Tween 80, etc.; and derivatives and polymers of such compounds. Of these, polysaccharides and polyethers are preferable, and polysaccharides are more preferable. Specifically, dextran, cellulose, Tween 20, Tween 40, Tween 60, and Tween 80 are preferably used. Further, a nonvolatile monomer and a nonvolatile water-soluble oligomer described in JP Patent Publication (Kokai) No. 2006-170832 A can also be used. Examples of such a nonvolatile monomer used herein may include: tetrose, pentose, heptose and hextose, wherein a hydroxyl group may be protected by a protecting group, and their glycoside; methyl glucoside; and cyclitols, wherein a hydroxyl group may be protected by a protecting group. Moreover, examples of such a nonvolatile water-soluble oligomer include: an oligomer represented by general formula (1) (-[CH2-CH(CONH2)-]n-), general formula (2) (-[CH2-CH2-O-]n-), or general formula (3) (-[CH2-CH(OH)-]n-) (wherein, in general formulas (1) to (3), n represents an integer between 10 and 200); and an oligosaccharide having an n number of sugars $(2 \le n \le 10)$, wherein a hydroxyl group may be protected by a protecting group. Furthermore, sugars described in US 2003/0175827 and DE 20306476A1, such as trehalose, sucrose, maltose, lactose, xylitol, fructose, mannitol, glucose, xylol, maltodextrin, saccharose, or polyvinylpyrrolidone may also be used. It is preferable that such compound S be substantially identical to the basic skeleton of a hydrophilic polymer used in the present invention. The term "basic skeleton" is used herein to mean a ring structure of sugar, for example. Although the type of a functional group or length differs, if such a ring structure is identical, it is considered that the basic skeleton is substantially identical.

With regard to the content of Compound S having a residue capable of forming hydrogen bond existing on a substrate, the ratio of the mean molecular weight of the aforementioned compound S to the mean molecular weight of a hydrophilic polymer is preferably between 0.005 and 0.2. If such a ratio is lower than the aforementioned range, compound S is likely to be crystallized. If such a ratio is higher than the aforementioned range, it is difficult for compound S to permeate into a hydrophilic polymer layer. When the aforementioned ratio is set within the aforementioned range, such problems are solved. Thereby, a higher effect of suppressing denaturation of physiologically active substances and a higher effect of suppressing aggregation can be obtained.

(5) Method of use of the solid substrate of the present invention

The solid substrate of the present invention to which a physiologically active substance is immobilized as described above can be used to detect and/or measure a substance which interacts with the physiologically active substance.

In the present invention, it is preferable to detect and/or measure an interaction between a physiologically active substance immobilized on the sensor substrate and a test substance by a nonelectric chemical method. Examples of a non-electrochemical method may include a surface plasmon resonance (SPR) measurement technique, a quartz crystal microbalance (QCM) measurement technique, and a measurement technique that uses functional surfaces ranging from gold colloid particles to ultra-fine particles.

Furthermore, the application range of a physiologically active substance that is inclusively immobilized on the solid substrate, which is obtained by the present invention, is not limited to sensors. The aforementioned physiologically active substance can be applied in many areas such as chemical reactions using a physiologically active substance immobilized on a substrate, production of useful substances, enzyme reactions in organic solvents, foods, medical treatments, affinity chromatography, waste liquid disposal, energy production, or genetic engineering.

In a preferred embodiment of the present invention, the solid substrate of the present invention can be used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.

If the wave number of a surface plasmon is determined from an incident angle causing the attenuated total reflection (ATR), that is, an attenuated total reflection angle ($\theta$SP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle ($\theta$SP) with nigh precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle ($\theta$SP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

When the solid substrate of the present invention is used in surface plasmon resonance analysis, it can be applied as a part of various surface plasmon, measurement devices described above.

The biosensor of the present invention can be used as a biosensor, which has a waveguide structure on the surface of a carrier, for example, and which detects refractive index changes using such a waveguide, In this case, the waveguide structure on the carrier surface has a diffraction grating, and in some cases, an additional layer. This waveguide structure is a planar waveguide body comprising a thin dielectric layer. Light gathered to the waveguide body form is introduced into such a thin layer by total internal reflection. The transmission velocity of the thus introduced light wave (hereinafter referred to as "mode") is indicated as a C/N value. Herein, C indicates the velocity of light in a vacuum, and N indicates an effective refractive index of the mode introduced into the waveguide body. Such an effective refractive index N is determined based on the structure of the waveguide body on one face, and is determined based on the refractive index of a medium adjacent to the thin waveguide layer on the other face. Conduction of a light wave is carried out not only in a thin planar layer, but also by another waveguide structure, and in particular, by a stripped waveguide body. In such a case, the waveguide structure is processed into the shape of a stripped film. It is an important factor for a biosensor that changes in effective refractive indexes N are generated as a result of changes in the medium adjacent to the waveguide layer, and changes in the refractive index and thickness of the waveguide layer itself or an additional layer adjacent to the waveguide layer.

The structure of a biosensor of this system is described in page 4, line 48 to page 14, line 15, and Figures 1 to 8 of JP Patent Publication (Kokoku) No. 6-27703 B (1994).

For example, in one embodiment, there is a structure whereby a waveguide layer comprising a planar thin layer is established on a substrate (e.g. Pyrex. (registered trademark) glass). A waveguide layer and a substrate form together a so-called waveguide body. Such a waveguide layer can be a multilayer laminated body such as an oxide layer ($SiO_2$, $SnO_2$, $Ta_2O_5$, $TiO_2$, $TiO_2$-$SiO_2$, $HfO_2$, $ZrO_2$, $Al_2O_3$, $Si_3N_4$, HfON. SiON, scandium oxide, or a mixture thereof) or a plastic layer (e.g. polystyrene, polyethylene, polycarbonate, etc.). For transmission of light into a waveguide layer as a result of total internal reflection, the refractive index of the waveguide layer must be greater than that of the adjacent medium (for example, a substrate, or an additional layer as described later). A diffraction grating is disposed on the surface of the waveguide layer or in the bosom thereof, which faces to a substrate or a measured substance. Such a diffraction grating can be formed in a carrier according to embossing, holography, or other methods. Subsequently, the upper surface of the diffraction grating is coated with a thin waveguide film having a higher refractive index. The diffraction grating has the functions to focus rays of light incident on the waveguide layer, to discharge the mode already introduced into the waveguide layer, or to transmit a portion of the mode in the travel direction and reflect a portion thereof. The grating area of the waveguide layer is covered with an additional layer. Such an additional layer can be a multilayer film, as necessary. This additional layer is able to have the function to carry out selective detection of a substance contained in a measured substance. In a preferred embodiment a layer having a detection function can be established on the outermost surface of such an additional layer. As such a layer having a detection function, a layer capable of immobilizing physiologically active substances can be used.

In another embodiment, it is also possible to adopt a structure whereby an array of diffraction grating waveguides is incorporated into wells of a microplate (JP Patent Publication (Kohyo) No. 2007-501432 A). That is to say, if such diffraction grating waveguides are aligned in the form of an array at the bottoms of wells of a microplate, the screening of a drug or chemical substance can be carried out at a high throughput.

In order to detect physiologically active substances existing on the upper layer (detection area) of a diffraction grating waveguide, the diffraction grating waveguide detects incident light and reflected light, so as to detect changes in refractive properties. For this purpose, one or more light sources (e.g. laser or diode) and one or more detectors (e.g. a spectrometer, a CCD camera, or other light detectors) can be used. As a method of measuring changes in refractive indexes, there are two different operational modes, namely, spectroscopy and an angle method. In spectroscopy, broadband beam used as incident light is transmitted to a diffraction grating waveguide, and reflected light is gathered, followed by a measurement with a spectrometer, for example. By observing the spectrum position of a resonant wavelength (peak), changes in refractive indexes on the surface of the diffraction grating waveguide or a periphery thereof, namely, a bond can be measured. On the other hand, in an angle method, light of a nominally single wavelength is gathered such that it generates a certain range of irradiation angle, and it is directed into the diffraction grating waveguide. The reflected light is measured with a CCD camera or other types of light detectors. By measuring the position of a resonance angle reflected by the diffraction grating wavelength, changes in refractive indexes on the surface of the diffraction grating waveguide or a periphery thereof, namely, a bond can be measured.

Still further, the solid substrate obtained by the present invention is not limited to a sensor. It can be applied in many areas such as chemical reactions using a physiologically active substance immobilized on a substrate, production of useful substances, enzyme reactions in organic solvents, foods, medical treatments, affinity chromatography, waste liquid disposal, energy production, or genetic engineering.

The present invention will be further specifically described in the following examples. However, the examples are not intended to limit the scope of the present invention.

Examples

Example 1: Production of substrates 1 and 2 and evaluation thereof

(1) Pretreatment of substrate and APS modification

A slide glass was immersed in 1 N NaOH aqueous solution overnight or longer. Thereafter, it was washed with ultrapure water, and it was then immersed in 1 N HCl, followed by washing with ultrasonic wave for 10 minutes. After completion of the washing with ultrasonic wave, the resultant was washed with ultrapure water, and it was then sufficiently washed by stirring. Liquid droplets were blown away by N2 blow.

(Preparation of 0.1 % 3-aminopropyltriethoxysilane (APS) solution)

0.1 g of APS was weighed, and it was then dissolved in a mixed solvent of ethanol/0.01 mol HCl (90/10), so as to prepare a 0.1 % APS solution.

The 0.1% APS solution was placed in an incubator at 60°C, and it was then preincubated for 15 minutes. The pretreated slide glass was placed in the 0.1% APS solution, and a reaction was carried out in the incubator at 60°C for 12 minutes.

After completion of the reaction, the reaction product was washed with a mixed solution (ethanol/ultrapure water = 9/1) contained in a disposable cup three times. After completion of the washing, $N_2$ blow was carried out, and the APS-modified slide glass was then subjected to a heat treatment in an incubator at 90°C for 3 hours.

(2) Active esterification of CMD (carboxymethyl dextran)

10 g of 1%-by-weight CMD (manufactured by Meito Sangyo Co., Ltd; mean molecular weight: 1,000,000; substitution degree: 0.59) solution was dissolved (carboxyl group amount: $5 \times 10^{-4}$ mol), and 10 ml of an aqueous solution that contained 1-ethyl-2,3-dimethylanminopropylcarbodiimide ($2 \times 10^{-5}$ M) and N-hydroxysuccinimide ($5 \times 10^{-5}$ M) was then added to the aforementioned solution, followed by stirring at room temperature for 1 hour.

(3) Binding reaction of CMD to substrate

1 ml of the active esterified CMD solution prepared in (2) above was added dropwise to the slide glass prepared in (1) above, and it was then fixed on the rotation center of an inner cup of a spin-coater (Model 408 (patented); manufactured by Nanotec Corporation) having a hermetically sealedinnercup. It was then spin-coated at 7000 rpm for 45 seconds, so as to form a thin film of active esterified carboxymethyl dextran on the substrate. A reaction was carried out at room temperature for 15 minutes, and the resultant was then immersed in 1 N NaOH aqueous solution for 30 minutes. Thereafter, it was washed with ultrapure water 5 times, so as to produce a CMD surface substrate. As a result of measurement by AFM, the film thickness in ultrapure water was found to be 200 nm.

(4) Modification of substrate with crosslinked hydrogel

150 μl of a 0.1 M sodium carbonate solution obtained by mixing EDC (0.4 M) with HODbht (3,4-dihydro-3-hydroxy-4-oxo-1,2,3-benzotriazine) (2.8M) at a ratio of 1/1 was added dropwise to a slide glass, to which CMD had bound, immediately after preparation of the aforementioned solution, In order to distribute the solution to the entire surface of the slide glass, a Sealon film was placed thereon. In order to prevent the slide glass from being dried, the slide glass was covered with a lid, and it was left at rest at room temperature for 5 minutes.

Thereafter, the resultant was washed with ultrapure water, and nitrogen blow was then performed. Subsequently, 150 μl of a 6 mM 2-aminoethyl methacrylate (0.1 M sodium carbonate aqueous solution) was added dropwise to the slide glass. A Sealon film was placed thereon, and it was then left at rest at room temperature for 5 minutes. Thereafter, the resultant was washed with ultrapure water, and nitrogen blow was then performed.

150 μl of ethanolamine (Biacore) was added dropwise to the slide glass, and a Sealon film was placed thereon, followed by leaving at rest at room temperature for 5 minutes. Thereafter, it was washed with ultrapure water, and N2 blow was then performed.

(4-2) Preparation of protein solution used in inclusive immobilization

300 μl of solution A (a mixed solution of 0.1 g of acrylamide (manufactured by Wako Pure Chemical Industries, Ltd.) and 2.2 g of a PBS buffer (pH 7.4)), 30 μl of solution B (2.5 mg/ml Avidin-FTTC (manufactured by SIGMA; pH 7.4)), and 6 μl of solution C (a mixed solution of 3 μl of 3-dimethylaminopropylnitrile (manufactured by Wako Pure Chemical Industries, Ltd.) and 177 μl of a PBS buffer) were mixed. Thereafter, nitrogen bubbling was carried out on the obtained mixed solution for 2 hours. Thereafter, 6 μl of solution D (obtained by adding 0.5 g of a PBS buffer to 3 mg of potassium persulfate (manufactured by Wako Pure Chemical Industries, Ltd.)) was added to the aforementioned mixed solution. The thus obtained mixed solution was defined as solution E.

(4-3) Formation of crosslinked hydrogel and immobilization of protein

80 μl of solution E was added dropwise onto a substrate, and it was then left at rest at a relative humidity of 100% at room temperature under light-shielded conditions for 1 hour. Thereafter, the substrate was washed with 100 μl of ultrapure water 10 times. The thus obtained substrate was defined as substrate 1.

The same operations as those for substrate 1 were carried out with the exception that a solution (solution F) obtained by substituting an acrylamide solution (solution A) with a PBS buffer was used instead of solution E. The thus obtained substrate was defined as substrate 2.

(5) Binding of analyte

A 0.1 mg/ml ATTO-680-Biotin (SIGMA) solution was allowed to come into contact with the aforementioned surface, on which a protein had been immobilized, and a reaction was carried out at room temperature under light-shielded conditions for 5 minutes. Thereafter, the resultant surface was washed with 100 μl of ultrapure water 10 times.

(6) Measurement of binding ability of Avidin-FITC immobilized on substrate

In terms of the two-dimensional fluorescent images at 473 nm and at 535 nm, substrate 1, substrate 2, and a background (of a protein-unmodined portion) were measured using FLA8000 (FUJIFILM Corporation). The fluorescence intensity of the background was subtracted from the fluorescence intensity of substrate 1 and that of substrate 2, so as to calculate the binding ability of Avidin-FITC immobilized on the substrate. The results are shown in Table 1.

Table 1:

|  | 473 nm | 535 nm |  |
| --- | --- | --- | --- |
| Substrate 1 | 11134 | 3330 | Example |
| Substrate2 | 1909 | 636 | Comparative example |

Both the fluorescence at 473 nm based on Avidin-FITC and the fluorescence at 53 5 nm based on ATrO-680-Biotin, observed from, substrate 1 that contained acrylamide, were approximately 5 times higher than those observed from substrate 2 that did not contain acrylamide. This result demonstrated that inclusive immobilization of Avidin-FITC could be carried out by the present method, and that the binding ability of the inclusively immobilized Avidin-FITC with biotin was not lost.

Example 2: Preparation of substrates 3 and 4 and evaluation thereof

(1) Pretreatment of substrate

In this experiment, a sensor chip Au of Biacore was used as a sensor chip surface, on which only a gold film was formed The sensor chip Au was treated with UV ozone for 12 minutes, and it was then immersed in a 1 mM 6-amino-1-octanethiol hydrochloride aqueous solution (manufactured by Dojindo Laboratories) at 40°C for 1 hour. Thereafter, the sensor chip was washed with ultrapure water 5 times.

(2) Active esterification of CMD (carboxymethyl dextran)

10 g of 1%-by-weight CMD (manufactured by Meito Sangyo Co., Ltd; mean molecular weight: 1,000,000; substitution degree: 0.59) solution was dissolved (carboxyl group amount: $5 \times 10^{-4}$ mol), and 10 ml of an aqueous solution that contained 1-ethyl-2,3-dimethylaminopropylcarbodiimide ($2 \times 10^{-5}$ M) and N-hydroxysuccinimide ($5 \times 10^{-5}$ M) was then added to the aforementioned solution, followed by stirring at room temperature for 1 hour.

(3) Binding reaction of CMD to substrate

1 ml of the active esterified CMD solution prepared in (2) above was added dropwise to the substrate prepared in (1) above, and it was then fixed on the rotation center of an inner cup of a spm-coatesr (Model 408 (patented); manufactured by Nanotec Corporation) having a hermetically sealed inner cup. It was then spin-coated at 7000 rpm for 45 seconds, so as to form a thin film of active esterified carboxymethyl dextran on the substrate having an amino group. A reaction was carried out at room temperature for 15 minutes, and the resultant was then immersed in a 1 N NaOH aqueous solution for 30 minutes. Thereafter, it was washed with ultrapure water 5 times, so as to produce a CMD surface substrate. As a result of measurement by AFM, the film thickness in ultrapure water was found to be 200 nm.

(4) Modification of substrate with crosslinked hydrogel

150 μl of a 0.1 M sodium carbonate solution obtained by mixing EDC (0.4 M) with HODbht (2.8M) at a ratio of 1/1 was added dropwise to a slide glass, to which CMD had bound, immediately after preparation of the aforementioned solution. In order to distribute the solution to the entire surface of the slide glass, a Sealon film was placed thereon. In order to prevent the slide glass from being dried, the slide glass was covered with a lid, and it was left at rest at room temperature for 5 minutes.

Thereafter, the resultant was washed with ultrapure water, and nitrogen blow was then performed. Subsequently, 150 µl of a 6 mM 2-aminoethyl methacrylate (0.1 M sodium carbonate aqueous solution) was added dropwise to the slide glass. A Sealon film was placed thereon, and it was then left at rest at room temperature for 5 minutes. Thereafter, the resultant was washed with ultrapure water, and nitrogen blow was then performed.

150 µl of ethanolamine (Biacore) was added dropwise to the slide glass, and a Sealon film was placed thereon, followed by leaving at rest at room temperature for 5 minutes. Thereafter, it was washed with ultrapure water, and N2 blow was then performed.

(4-2) Preparation of protein solution used in inclusive immobilization

300 µl of solution A (a mixed solution of 0.1 g of acrylamide (manufactured by Wako Pure Chemical Industries, Ltd.) and 2.2 g of a PBS buffer (pH 7.4)), 30 µl of solution B (2.5 mg/ml Avidin-FITC (manufactured by SIGMA; pH 7.4)), and 6 µl of solution C (a mixed solution of 3 µl of 3-dimethylaminopropylnitrile (manufactured by Wako Pure Chemical Industries, Ltd.) and 177 µl of a PBS buffer) were mixed. Thereafter, nitrogen bubbling was carried out on the obtained mixed solution for 2 hours. Thereafter, 6 µl of solution D (obtained by adding 0.5 g of a PBS buffer to 3 mg of potassium persulfate (manufactured by Wako Pure Chemical Industries, Ltd)) was added to the aforementioned mixed solution. The thus obtained mixed solution was defined as solution E.

(4-3) Formation of crosslinked hydrogel and immobilization of protein

80 µl of solution E was added dropwise onto a substrate, and it was then left at rest at a relative humidity of 100% at room temperature under light-shielded conditions for 1 hour. Thereafter, the substrate was washed with 100 µl of ultrapure water 10 times. The thus obtained substrate was defined as substrate 3.

The same operations as those for substrate 3 were carried out with the exception that a solution (solution F) obtained by substituting an acrylamide solution (solution A) with a PBS buffer was used instead of solution E. The thus obtained substrate was defined as substrate 4.

(5) Biacore measurement

Substrate 3 was set in Biacore 3000 (manufactured by Biacore). HBS-N was used as a running buffer at a flow rate of 10 µl/min in the experiment. When 0.01 mg/ml ATTO 680 Biotin (molecular weight: 836) was allowed to come into contact with the substrate for 5 minutes, the amount of the aforementioned substance bound to the substrate was found to be 380 RU.

Substrate 4 was set in Biacore 3000 (manufactured by Biacore). HBS-N was used as a running buffer at a flow rate of 10 µl/min in the experiment. When 0.1 mg/ml Biotin HRP (molecular weight: 40,000) was allowed to come into contact with the substrate for 5 minutes, the amount of the aforementioned substance bound to the substrate was found to be 185 RU.

Thus, when ATTO 680 Biotin (molecular weight 836) that was a low molecular weight derivative of Biotin was used, the amount of the substance bound that was 380 RU was observed. On the other hand, when Biotin HRP (molecular weight: 40,000) that was a protein derivative of Biotin was used, the observed amount of the substance bound was only 185 RU. This result demonstrates that low molecular weight substances are able to penetrate into the network structure that inclusively immobilizes proteins, but that proteins are not able to penetrate into the aforementioned network structure. The aforementioned results are shown in Table 2.

Table 2:

|  | Molecular weight | Immobilized amount (RU) | Molar ratio |
|---|---|---|---|
| Avidin-FITC | 48000 | 5780 | 1.0 |
| ATTO 680 Biotin | 836 | 380 | 3.7 |
| Biotin HRP | 40000 | 185 | 0.05 |

**Claims**

1. A solid substrate, which has a physiologically active substance in a crosslinked hydrogel composed of a polysaccharide that was covalently bound to the surface of the solid substrate.

**2.** A solid substrate, wherein a physiologically active substance is inclusively immobilized in a crosslinked hydrogel composed of a polysaccharide which was covalently bound to the surface of the solid substrate.

**3.** The solid substrate according to claim 1, wherein the crosslinked hydrogel is formed using a polysaccharide having a double bond or a derivative thereof, a water-soluble monomer, and a water-soluble initiator.

**4.** The solid substrate according to claim 1, which is produced by allowing an aqueous solution comprising a physiologically active substance, a water-soluble monomer and a water-soluble initiator, to come into contact with the solid substrate, to which a polysaccharide having a double bond or a derivative thereof was bound, so as to carry out polymerization.

**5.** The solid substrate according to claim 4, wherein, in the aqueous solution comprising a physiologically active substance, a water-soluble monomer and a water-soluble initiator, the concentration of the water-soluble monomer is between 100 mM and 1 M.

**6.** The solid substrate according to claim 4, wherein, in the aqueous solution comprising a physiologically active substance, a water-soluble monomer and a water-soluble initiator, the concentration of the water-soluble initiator is between 0.1 mM and 2 mM.

**7.** The solid substrate according to claim 4, wherein the water-soluble initiator is persulfate.

**8.** The solid substrate according to claim 4, wherein the water-soluble monomer has an amide structure or a polyether structure.

**9.** The solid substrate according to claim 4, wherein the aqueous solution comprising a physiologically active substance, a water-soluble monomer and a water-soluble initiator, further comprises a polymerization promoter.

**10.** The solid substrate according to claim 9, wherein the polymerization initiator is β-dimetbylaminvpropionitrile, N,N, N',N'-tetramethylemylenediamine, or sodium sulfite.

**11.** The solid substrate according to claim 4, wherein the polysaccharide having a double bond or a derivative thereof is generated as a result of the reaction of a carboxyl group-containing polysaccharide which was covalently bound to the solid substrate or a derivative thereof with an amine having a double bond.

**12.** The solid substrate according to claims 1, wherein the physiologically active substance is a protein.

**13.** A biosensor, which comprises the solid substrate of claim 1.

**14.** The biosensor according to claim 13, which is used in surface plasmon resonance analysis.

**15.** A method for producing a solid substrate having a physiologically active substance in a crosslinked hydrogel composed of a polysaccharide that was covalently bound to the surface of the solid substrate, which comprises allowing an aqueous solution comprising a physiologically active substance, a water-soluble monomer and a water-soluble initiator, to come into contact with a solid substrate, to the surface of which a polysaccharide having a double bond or a derivative thereof was bound, so as to carry out polymerization.

**16.** The method for producing a solid substrate according to claim 15, wherein the polysaccharide having a double bond or a derivative thereof that was bound to the surface of the solid substrate has a film thickness in water of between 5 nm and 500 nm.

**European Patent Office**

## EUROPEAN SEARCH REPORT

Application Number

EP 08 00 5791

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 436 161 A (BERGSTROEM JAN [SE] ET AL) 25 July 1995 (1995-07-25) <br> * claims 1-15 * <br> * column 5, line 20 - column 8, line 48 * <br> ----- | 1-16 | INV. <br> G01N33/543 <br> G01N33/553 <br> G01N21/55 |
| X | WO 00/65352 A (EIDGENOSSISCH TECH HOCHSCHULE [CH]; HUBBELL JEFFREY A [CH]) 2 November 2000 (2000-11-02) <br> * page 6, line 28 - page 8, line 30 * <br> * page 15, line 9 - page 18, line 2 * <br> * page 24, line 24 - page 30, line 7 * <br> * page 44, line 4 - page 49, line 31 * <br> * figures 2A,2B * <br> ----- | 1-16 | |
| X | US 5 624 711 A (SUNDBERG STEVEN A [US] ET AL) 29 April 1997 (1997-04-29) <br> * column 5, lines 25-35 * <br> * column 13, line 45 - column 17, line 40 * <br> * figures 6-11; examples 6-9 * <br> ----- | 1-16 | |
| X | EP 1 696 235 A (FUJI PHOTO FILM CO LTD [JP]) 30 August 2006 (2006-08-30) <br> * the whole document * <br> ----- | 1-16 | TECHNICAL FIELDS SEARCHED (IPC) <br><br> G01N |
| X | LOEFAAS S ET AL: "A NOVEL HYDROGEL MATRIX ON GOLD SURFACES IN SURFACE PLASMON RESONANCE SENSORS FOR FAST AND EFFICIENT COVALENT IMMOBILIZATION OF LIGANDS" JOURNAL OF THE CHEMICAL SOCIETY, CHEMICAL COMMUNICATIONS, CHEMICAL SOCIETY. LETCHWORTH, GB, no. 21, 1 January 1990 (1990-01-01), pages 1526-1528, XP008050238 ISSN: 0022-4936 * the whole document * <br> ----- | 1-16 | |

The present search report has been drawn up for all claims

| Place of search | Date of completion of the search | Examiner |
|---|---|---|
| The Hague | 26 June 2008 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 00 5791

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2008

| Patent document cited in search report | | Publication date | Patent family member(s) | | Publication date |
|---|---|---|---|---|---|
| US 5436161 | A | 25-07-1995 | NONE | | |
| WO 0065352 | A | 02-11-2000 | AU | 769571 B2 | 29-01-2004 |
| | | | AU | 4684600 A | 10-11-2000 |
| | | | CA | 2371011 A1 | 02-11-2000 |
| | | | EP | 1190252 A1 | 27-03-2002 |
| | | | JP | 2003516519 T | 13-05-2003 |
| US 5624711 | A | 29-04-1997 | NONE | | |
| EP 1696235 | A | 30-08-2006 | US | 2006240478 A1 | 26-10-2006 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2815120 B
- JP 5133928 A
- JP Z7260107 B
- JP 2006170832 A
- US 20030175827 A
- DE 20306476 A1

- JP 6167443 A
- JP 11326194 A
- JP 2000398309 A
- JP 2001330560 A
- JP 6027703 B
- JP 2007501432 A

**Non-patent literature cited in the description**

- **WHITESIDES et al.** Details of the method. *Chemical Review,* 2005, vol. 105, 1103-1169

- **PROFESSOR GRZYBOWSKI.** *Curr. Org. Chem.,* 2004, vol. 8, 1763-1797
- *Bunko Kenkyu,* 1998, vol. 47 (1), 21-2326-27